# EUROPÄISCHE PATENTSCHRIFT

(11) **EP 0 472 560 B1**
(45) Veröffentlichungstag und Bekanntmachung des Hinweises auf die Patenterteilung: **10.07.1996**
(21) Anmeldenummer: 90907005.4
(22) Anmeldetag: 07.05.1990
(51) Int. Cl.: C07D 263/14, C07D 265/08, C08K 5/35

(54) **CYCLISCHE POLYIMINOETHER**
CYCLIC POLYIMINOETHERS
POLYIMINOETHERS CYCLIQUES

(30) Priorität: 16.05.1989 DE 3915874
(43) Veröffentlichungstag der Anmeldung: 04.03.1992
(73) Patentinhaber: Henkel Kommanditgesellschaft auf Aktien, 40191 Düsseldorf (DE)
(72) Erfinder: HASE, Brigitte, D-4006 Erkrath 1 (DE); KRAUSE, Horst-Jürgen, D-4000 Düsseldorf 13 (DE); FRISTAD, William, Santa Rosa, CA 95404 (US)
(86) Internationale Anmeldenummer: EP9000733
(87) Internationale Veröffentlichungsnummer: WO9014341

(56) Entgegenhaltungen:
- EP-A- 0 273 368
- DE-A- 2 029 524
- US-A- 3 763 177
- US-A- 4 378 357

## Beschreibung

Die vorliegende Erfindung betrifft neue Verbindungen, die zwei-, drei- oder viermal im Molekül eine fünf- oder sechsgliedrige cyclische Iminoethergruppe enthalten, ein Verfahren zu ihrer Herstellung und ihre Verwendung.

Cyclische Polyiminoether sind als wertvolle Zwischenprodukte bekannt und werden beispielweise gemäß EP 273 368, DE 20 29 524 und DE 35 39 593 zur Herstellung von Kunststoffen, Harzen und Klebemassen eingesetzt. Einer breiten Anwendung dieser Zwischenprodukte stand bisher allerdings entgegen, daß ein einfaches und zugleich zu guten Ausbeuten führendes Syntheseverfahren für Polyiminoether fehlte.

Es wurde nun ein Verfahren zur Herstellung einer neuen Gruppe von cyclischen Polyiminoethern gefunden, das von gut zugänglichen Ausgangsmaterialien ausgeht und hohe Ausbeuten liefert.

Gegenstand der Erfindung ist daher ein Verfahren zur Herstellung von Polyiminoethern der allgemeinen Formel I 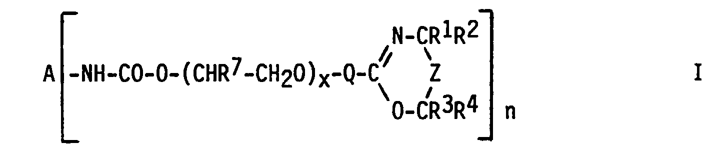 in der A eine aliphatische, aromatische oder araliphatische Gruppe mit 4 bis 40 C-Atomen und n freien Valenzen an verschiedenen C-Atomen darstellt, x einen Wert zwischen 0 und 40 aufweist, n einen der Werte 2, 3 oder 4 hat, Z eine direkte Bindung oder die Gruppe CR⁵R⁶ bedeutet, die Reste R¹ bis R⁶ jeweils unabhängig voneinander H, Alkyl oder Aryl mit bis zu 8 C-Atomen bedeuten, R⁷ ausgewählt ist aus der Gruppe Wasserstoff, Methyl und Ethyl, und Q eine Phenylengruppe, die durch 1 oder 2 Methylgruppen substituiert sein kann, oder die Gruppe -CHR-M- bedeutet, wobei R ausgewählt ist aus der Gruppe H, Alkyl oder Alkenyl und M ausgewählt ist aus der Gruppe Alkylen, Alkenylen, Arylen und Alkarylen, und die Molekülteile R und M jeweils unabhängig voneinander 0 bis 12 C-Atome aufweisen und durch eine Alkoxygruppe mit 1 bis 3 C-Atomen substituiert sein können, dadurch gekennzeichnet, daß ein Polyisocyanat der Formel A(-NCO)ₙ (II) mit einer wenigstens äquivalenten Menge eines Iminoethers der Formel III 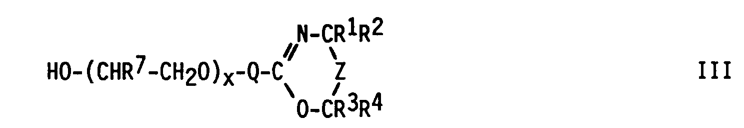 wobei A, Q, R¹ bis R⁷, Z, x und n die oben angegebene Bedeutung haben, unter den für die Umsetzung von Alkoholen mit Isocyanaten üblichen Bedingungen, gegebenenfalls unter Zusatz eines üblichen Katalysators und/oder Lösungsmittels, umgesetzt wird.

Die neuen Polyiminoether lassen sich in praktisch quantitativer Ausbeute aus Polyisocyanaten der Formel II durch Umsetzung mit Iminoethern der Formel III synthetisieren, wobei durch Reaktion der Hydroxylgruppen aus III mit den Isocyanatgruppen in II in einer Stufe die neuen Polyiminoether gebildet werden.

Der glatte Verlauf dieser Reaktion ist um so erstaunlicher, als bekannt ist, daß in 2-Stellung substituierte cyclische Iminoether gegenüber Isocyanaten als bifunktionelle Reagentien unter exocyclischer Verschiebung der Doppelbindung am Iminoetherring reagieren (s. beispielsweise R. Nehring et al, Liebigs Ann. Chem. 698, 167 (1966)). Statt dieser Reaktion, die bei Anwendung von Polyisocyanaten der Formel II auf cyclische Iminoether zu hochmolekularen Produkten führen müßte, werden im vorliegenden Falle definierte niedermolekulare Polyiminoether der Formel I in praktisch quantitativer Ausbeute erhalten. Sofern nicht mehr als die äquivalente Menge an Isocyanat, bezogen auf Hydroxylgruppen, verwendet wird, werden selbst zusätzlich anwesende hydroxylgruppenfreie cyclische Iminoether praktisch nicht angegriffen.

Die Reaktion zwischen den Isocyanaten II und den Iminoethern III verläuft glatt bei den für die Umsetzung von Alkoholen mit Isocyanaten üblichen Bedingungen, das heißt bei Temperaturen zwischen etwa Raumtemperatur und etwa 160 °C und Reaktionszeiten von etwa 1 bis 10 Stunden. Je nach Reaktivität der Isocyanate und der Alkoholkomponente können diese Bedingungen aber auch deutlich über- oder unterschritten werden. Die Reaktion läßt sich in Anwesenheit geeigneter aprotischer Lösungsmittel, beispielsweise aromatische Kohlenwasserstoffe, Chlorkohlenwasserstoffe, Ester oder Ketone, oder auch lösungsmittelfrei durchführen, wobei im allgemeinen unter Auschluß von Feuchtigkeit gearbeitet wird. Zur Beschleunigung können die für die Umsetzung von Alkoholen mit Isocyanaten üblichen Katalysatoren, vor allem Zinnverbindungen wie Dibutylzinndilaurat oder tertiäre Amine wie 1,4-Diazabicyclo-[2,2,2]-octan zugesetzt werden. Der Fortgang der Reaktion läßt sich durch Bestimmung des noch nicht umgesetzten Isocyanats, beispielsweise durch Umsetzung mit überschüssigem Dibutylamin in Toluol und Rücktitration mit HCl, verfolgen. Da die Reaktion auch bei Verwendung äquivalenter Mengen der Reaktionspartner, d. h. n Mol Iminoether (III) für ein Mol Isocyanat (II), quantitativ verläuft, gestaltet sich die Aufarbeitung der Syntheseansätze äußerst einfach oder entfällt ganz.

Bevorzugt werden im Rahmen der vorliegenden Erfindung cyclische Polyiminoether der Formel Ia hergestellt 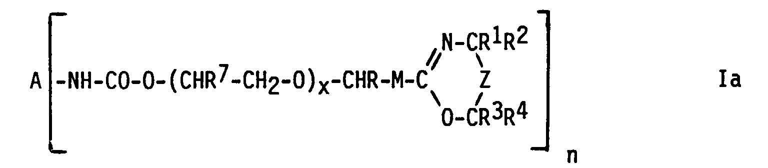 die aus Polyisocyanaten (II) und n Mol Iminoethern der Formel IIIa 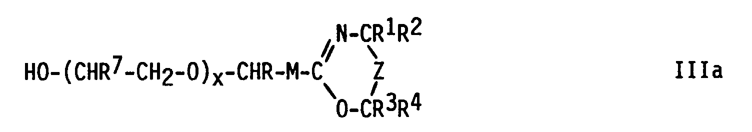 zugänglich sind. In diesen Formeln haben R und M ebenfalls die schon oben angegebene Bedeutung.

Weiterhin werden bevorzugt die Bisiminoether (I, n = 2), die aus Diisocyanaten zugänglich sind, hergestellt, wobei wiederum die Herstellung aus 1,6-Hexamethylendiisocyanat; 2,4-Toluylendiisocyanat; 4,4'- und/oder 2,4'-Diphenylmethandiisocyanat, 4,4'-Dicyclohexylmethandiisocyanat, m-Tetramethylxylolylendiisocyanat und Isophorondiisocyanat besonders bevorzugt wird.

Die als Ausgangsmaterial dienenden Di- und Polyisocyanate der Formel II sind in der chemischen Technik wohlbekannte Verbindungen, die üblicherweise aus den entsprechenden Aminen mit COCl₂ hergestellt werden. Sie enthalten im Molekülteil A vorzugsweise 6 - 26 C-Atome. Neben den oben erwähnten Diisocyanaten seien beispielsweise N,N',N"-Tris-(omega-isocyanatohexyl)-biuret und 2,2,4-Trimethylhexamethylen-1,6-diisocyanat erwähnt. Im übrigen wird hierzu auf die Angaben in Handbüchern wie Kirk-Othmer, Encyclopedia of Chemical Technology, 3. Auflage, Band 23, S. 586 (1984), hingewiesen.

Die erfindungsgemäß benötigten Iminoether (III) sind ebenfalls in an sich bekannter Weise, etwa durch Cyclisierung von Derivaten der Hydroxycarbonsäuren HO-CHR-M-CO₂H, beispielsweise gemäß Verfahren der älteren Patentanmeldungen EP 8811090.5, DE-P 39 14 133, DE-P 39 14 155 und DE-P 39 14 159 oder gemäß dem Verfahren von Litt und Levy (J. Polym. Sci. Al, 6, 1883 (1968) oder anderen üblichen Syntheseverfahren für cyclische Iminoether (z. B. DE 1 445 642 oder S. Kobayaski und T. Saegusa in "Ring-Opening Polymerization" Vol. 2, London 1984, Seite 762 folgende) gut zugänglich. Iminoether (III), in denen x einen Wert größer 0 aufweist, können aus Derivaten von Hydroxycarbonsäuren der Formel HO-(CHR⁷-CH₂-O-)ₓ-CHR-M-CO₂H gewonnen werden, die ihrerseits in bekannter Weise beispielsweise durch Umsetzung der Derivate von Hydroxycarbonsäuren HO-CHR-M-CO₂H mit x Mol Butylenoxid, Propylenoxid oder, vorzugsweise, Ethylenoxid zugänglich sind. Es ist aber auch möglich, diese Iminoether herzustellen, indem man zunächst Iminoether der Formel III mit x = 0 wie oben angegeben herstellt und diese dann in üblicher Weise mit x Mol Butylenoxid, Propylenoxid oder, vorzugsweise, Ethylenoxid alkoxyliert. Vorzugsweise hat x Werte von 0 bis 10, insbesondere 1 oder 0. Besonders bevorzugt werden dabei die Iminoether, die von Ricinolsäure, Dihydroricinolsäure, Caprolacton oder von epoxidierten und mit einwertigen niederen Alkoholen ringgeöffneten ungesättigten Fettsäuren abgeleitet sind. Im ersten Fall besteht der 2-Alkylrest des cyclischen Iminoethers III aus dem 11-Hydroxy-8-heptadecenylrest, im zweiten Fall aus dem 11-Hydroxyheptadecylrest, im dritten Fall aus dem 5-Hydroxypentylrest und im letzten Fall vorzugsweise aus einem geradkettigen 17 oder 21 C-Atome enthaltenden Alkylrest, der innenständig vicinal durch eine Hydroxyl- und eine Alkoxylgruppe (mit vorzugsweise 1 bis 3 C-Atomen) substituiert ist.

Die neuen Polyiminoether sind vielseitig verwendbare Zwischenprodukte. Ein besonders vorteilhaftes Einsatzgebiet sind Verfahren zur Herstellung von gegebenenfalls vernetzten Polymeren, wobei die Polyiminoether allein, vorzugsweise aber zusammen mit anderen niedermolekularen oder polymeren Ausgangsmaterialien eingesetzt werden. Die neuen Polyiminoether der Formel I und deren Verwendung sind daher ebenfalls Gegenstand der Erfindung.

Beispiele für die Verwendung der erfindungsgemäßen Bis-, Tris- und Tetrakisiminoether sind die vernetzende Polymerisation mit Polycarbonsäuren gemäß DE 35 39 593 oder die Herstellung von Harzen durch Reaktion mit mehrfunktionellen Alkoholen oder Aminen gemäß EP 273 368.

Bei den beanspruchten Polyiminoethern der Fomrel I kann x Werte zwischen 0 und 10 annehmen, mit der Maßgabe, daß wenigstens 4 von den Resten R¹ bis R⁶ Wasserstoffatome sind und die übrigen aus C₁-C₃-Alkyl bestehen.

Bevorzugt werden im Rahmen der vorliegenden Erfindung cyclische Polyiminoether der Fomel Ia, die aus Polyisocyanaten (II) und n Mol Iminoethern der Formel IIIa hergestellt werden. In diesen Formeln haben R und M ebenfalls die schon oben angegebene Bedeutung.

Weiterhin bevorzugt sind die aus 2-(Hydroxyalkyl)-Δ²-oxazolinen (III, Z = direkte Bindung) hergestellten Polyimoether. Von diesen wiederum werden diejenigen mit den folgenden Substitutionsmustern besonders bevorzugt:
R¹ = CH₃, R²-R⁴ = H;
R¹ und R² = CH₃, R³ und R⁴ = H;
R³ = CH₃ oder C₂H₅; R¹, R² und R⁴ = H.

Ganz besonders bevorzugt werden schließlich diejenigen Polyiminoether, in denen Z die Methylengruppe oder eine direkte Bindung und alle Reste R¹ - R⁴ Wasserstoffe darstellen.

Weiterhin bevorzugt sind die Bisiminoether (I, n = 2), die mit Diisocyanaten gebildet werden, wobei wiederum die aus 1,6-Hexamethylendiisocyanat; 2,4-Toluylendiisocyanat; 4,4'- und/oder 2,4'-Diphenylmethandiisocyanat, 4,4'-Dicyclohexylmethandiisocyanat, m-Tetramethylxylolylendiisocyanat und Isophorondiisocyanat hergestellten Bisiminoether besonders bevorzugt werden.

### BEISPIELE

1. Unter Feuchtigkeitsausschluß wurde eine Mischung von 323 g (1 Mol) 2-(11-Hydroxy-8-heptadecenyl)-2-oxazolin (technisch, aus Ricinolsäure) und 125 g (0,5 Mol) 4,4'-Diphenylmethandiisocyanat (Desmodur ^{(R)} M, Firma Bayer, Leverkusen) 4 Stunden auf 80 °C erhitzt. Nach dieser Zeit war durch Titration mit Dibutylamin kein Isocyanat mehr nachweisbar. Das Produkt lag als schwach gelbliche viskose Flüssigkeit vor.

Die folgende Struktur des Produktes wurde durch das H-NMR-Spektrum gesichert: 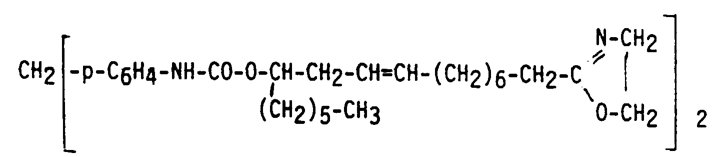
NMR (1 % in CDCl₃, delta-Skala)
0,8 - 1,0 ppm (t; 6 H)
1,1 - 2,4 ppm (5m; ca. 52 H)
3,7 - 3,9 ppm (t + s; 6 H)
4,1 - 4,3 ppm (t; 4 H)
4,7 - 4,9 ppm (q; 2 H)
5,2 - 5,6 ppm (m; 4 H)
6,6 - 6,7 ppm (s; 2 H)
7,0 - 7,4 ppm (2 d; 8 H)

2. Unter Feuchtigkeitsausschluß wurde eine Mischung von 323 g (1 Mol) 2-(11-Hydroxy-8-heptadecenyl)-2-oxazolin (technisch) und 84 g (0,5 Mol) 1,6-Hexamethylendiisocyanat (Desmodur ^{(R)} H, Bayer) 10 Stunden auf 80 °C erhitzt. Nach dieser Zeit war kein Isocyanat mehr nachweisbar. Das Produkt erstarrte beim Abkühlen zu einem wachsartigen Feststoff. Die folgende Struktur des Produktes wurde durch das H-NMR-Spektrum belegt: 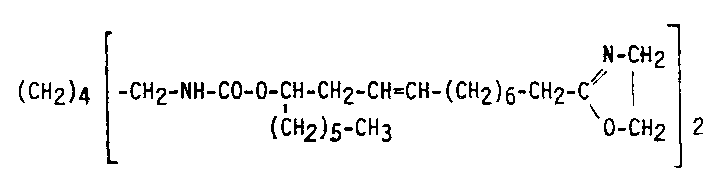
NMR (1 % in CDCl₃, delta-Skala)
0,8 - 1,0 ppm (t; 6 H)
1,1 - 2,4 ppm (5 m, ca. 60 H)
3,0 - 3,3 ppm (q; 4 H)
3,7 - 3,9 ppm (t; 4 H)
4,1 - 4,3 ppm (t; 4 H)
4,5 - 4,9 ppm (t + s; 4 H)
5,2 - 5,6 ppm (m; 4 H)

3. Unter Feuchtigkeitsausschluß wurden 157g (1 Mol) 2-(5-Hydroxypentyl)-2-oxazolin und 125 g (0,5 Mol) 4,4'-Diphenylmethandiisocyanat (Desmodur ^{(R)} M, Bayer) unter Rühren bis zur vollständigen Umsetzung für 4 Stunden auf 80 °C erhitzt. Das Produkt erstarrte beim Abkühlen zu einem Feststoff. Die Struktur des Produktes ergab sich aus dem H-NMR-Spektrum. 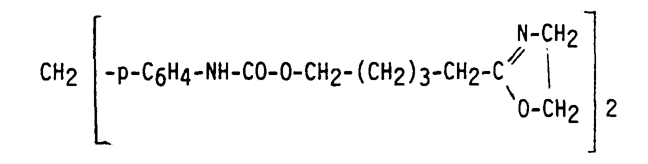
NMR (1 % in CDCl₃, delta-Skala)
1,3 - 1,8 ppm (2 m; ca. 12 H)
2,2 - 2,4 ppm (t; 4 H)
3,7 - 4,0 ppm (t + s; 6 H)
4,0 - 4,3 ppm (2 t; 8 H)
6,9 - 7,0 ppm (s; 2 H)
7,0 - 7,4 ppm (2 d; 8 H)

4. Unter Feuchtigkeitsausschluß wurden 411 g (1 Mol) 2-[12,13-(Hydroxy,methoxy)-heneicosyl]-2-oxazolin (Derivat der epoxidierten und mit Methanol ringgeöffneten Erucasäure, technisch) und 125 g (0,5 Mol) 4,4'-Diphenylmethandiisocyanat (Desmodur ^{(R)} M,) innig gemischt und dann für 4 Stunden auf 80 °C erhitzt. Nach dieser Zeit war kein Isocyanat mehr nachweisbar.

Das Produkt lag nach dem Abkühlen als gelbliche, hochviskose Flüssigkeit vor.

Die Struktur des Produktes wurde durch das H-NMR-Spektrum belegt: 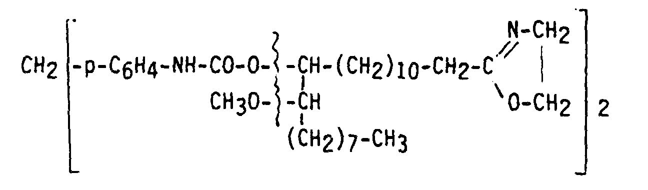
NMR (1 % in CDCl₃,delta-Skala)
0,8 - 1,0 ppm (t, 6 H)
1,1 - 1,7 ppm (3m, ca. 68 H)
2,2 - 2,4 ppm (t, 4 H)
3,1 - 3,3 ppm (m, 2 H)
3,3 - 3,5 ppm (s, 6 H)
3,7 - 3,9 ppm (t + s, 6 H)
4,1 - 4,3 ppm (t, 4 H)
4,8 - 5,0 ppm (m, 2 H)
6,7 - 6,9 ppm (2s, 2 H)
7,0 - 7,4 ppm (2d, 8 H)

5. Unter Feuchtigkeitsausschluß wurden 323 g (1 Mol) 2-(11-Hydroxy-8-heptadecenyl)-2-oxazolin (technisch) und 158 g (0,33 Mol) N,N',N"-Tris-(omega-isocyanatohexyl)-biuret (Desmodur ^{(R)} N) 8 Stunden bei 80 °C gerührt. Nach dieser Zeit war kein Isocyanat mehr nachweisbar. Das Produkt lag nach dem Abkühlen als hellgelbe viskose Flüssigkeit vor.
Die Struktur des Produktes wurde durch das H-NMR-Spektrum belegt. 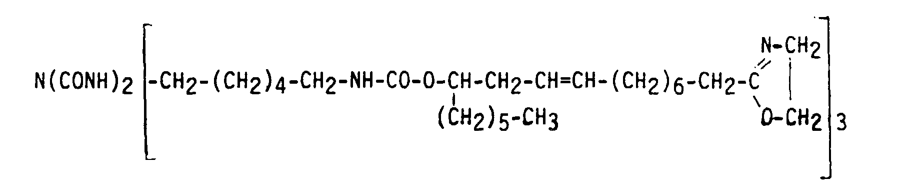
NMR(1 %ig in CDCl₃, delta-Skala)
0,8 - 1,0 ppm (t; 9H)
1,1 - 2,4 ppm (5m; ca. 102 H)
3,0 - 3,4 ppm (2m; 10 H)
3,6 - 3,9 ppm (m + t; 2 + 6 H)
4,1 - 4,3 ppm (t; 6 H)
4,6 - 4,9 ppm (m; 6 H)
5,2 - 5,6 ppm (m; 6 H)
7,1 - 7,6 ppm (m, 2 H)

6. Unter Feuchtigkeitsausschluß wurden 325 g (1 Mol) 2-(11-Hydroxyheptadecyl)-2-oxazolin (technisch) und 87 g (0,5 Mol) 2,4-Toluylendiisocyanat (Desmodur ^{(R)} T,) 12 Stunden bei 80 °C gerührt. Nach dieser Zeit war kein Isocyanat mehr nachweisbar. Das Produkt lag als gelbliche, viskose Flüssigkeit vor. Die Struktur des Produktes wurde durch das H-NMR-Sepktrum belegt: 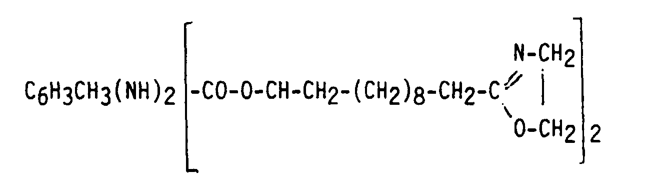
NMR (1 %ig in CDCl₃, delta-Skala)
0,8 - 1,0 ppm (t; 6 H)
1,1 - 1,7 ppm (2m; ca. 56 H)
2,1 - 2,4 ppm (s + t; 3 + 4 H)
3,7 - 3,9 ppm (t; 4 H)
4,1 - 4,3 ppm (t; 4 H)
4,7 - 4,9 ppm (m; 2 H)
6,4 ppm (s; 1 H)
6,7 ppm (s; 1 H)
7,0 - 7,4 ppm (2d; 2 H)
7,8 ppm (s; 1 H)

7. Unter Feuchtigkeitsausschluß wurden 337 g (1 Mol) 2-(11-Hydroxy-8-heptadecenyl)-2-oxazin (technisch, hergestellt gemäß älterer Patentanmeldung DE P 39 14 155) mit 125 g (0,5 Mol) 4,4-Diphenylmethan-diisoyanat (Desmodur ^{(R)} M, Bayer) 5 Stunden bei 80 °C gerührt. Nach dieser Zeit war kein Isocyanat mehr nachweisbar. Das Produkt lag als gelbliche, viskose Flüssigkeit vor. Die Struktur des Produktes wurde durch das H-NMR-Sepktrum belegt: 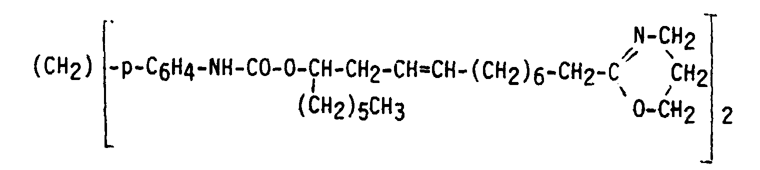
NMR (1 %ig in CDCl₃, delta-Skala)
0,8 - 1,0 ppm (t; 6 H)
1,1 - 2,4 ppm (5m; ca. 56 H)
3,2 - 3,4 ppm (t; 4 H)
3,8 - 3,9 ppm (s; 2 H)
4,0 - 4,3 ppm (t; 4 H)
4,7 - 5,0 ppm (m; 2 H)
5,2 - 5,6 ppm (m; 4 H)
6,8 ppm (s; 2 H)
7,0 - 7,4 ppm (2d; 8 H)

8. Unter Feuchtigkeitsanschluß wurden 323 g (1 Mol) 2-(11-Hydroxy-8-heptadecenyl)-2-oxazolin (technisch) und 111 g (0,5 Mol) Isophorondiisocyanat (VEBA-Chemie) 8 Stunden bei 80 °C gerührt. Nach dieser Zeit war kein Isocyanat mehr nachweisbar. Das Produkt lag als hellgelbe, viskose Flüssigkeit vor. Die Struktur des Produktes wurde durch das H-NMR-Sektrum belegt: 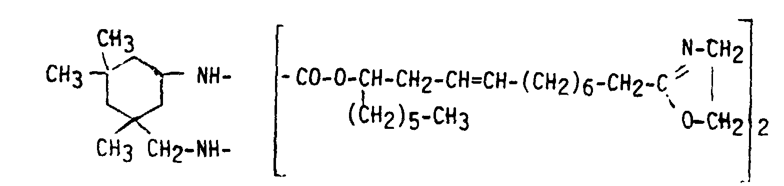
NMR (1 %ig in CDCl₃, delta-Skala)
0,8 - 1,9 ppm (6m, ca. 61 H)
1,9 - 2,4 ppm (2m; 4 + 8 H)
2,8 - 3,0 ppm (m; 2 H)
3,2 - 3,3 ppm (m; 1 H)
3,7 - 3,9 ppm (t; 4 H)
4,1 - 4,3 ppm (m; 4 H)
4,3 - 4,5 ppm (m; ca. 2 H)
4,6 - 4,9 ppm (m; 2 H)
5,2 - 5,6 ppm (m; 4 H)

9. Unter Stickstoff wurden 41,4 g (0,2 Mol) 2-(p-Hydroxyethoxyphenyl)-2-oxazolin (Fp: 146 °C, hergestellt aus p-(2-Hydroxyethoxy)-benzoesäure (Beilstein E II 10, S. 93) und Ethanolamin nach dem Verfahren, beschrieben in der älteren deutschen Patentanmeldung P 39 14 133) mit 26,2 g (0,1 Mol) Methylen-bis(4-cyclohexylisocyanat) (Desmodur ^{(R)} W, Bayer) bei Raumtemperatur gemischt und für 5 Stunden auf 180 °C erhitzt. Nach dieser Zeit war kein Isocyanat mehr nachweisbar. Das Produkt erstarrte beim Abkühlen zu einem gelblichen, wachsartigen Feststoff.
Die Struktur des Produktes wurde durch das H-NMR-Spektrum belegt: 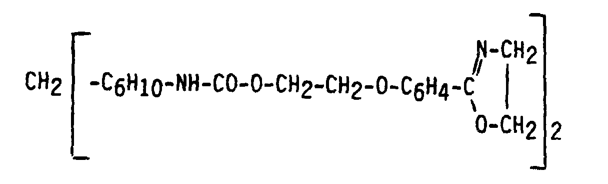
NMR (1 % in CDCl₃, delta-Skala)
0,8 - 2,1 ppm (4m; 20 H)
3,3 - 3,9 ppm (2m; 2 H)
3,9 - 4,6 ppm (3m; 16 H)
4,6 - 5,1 ppm (2d; 2 H)
6,8 - 7,0 ppm (m; 4 H)
7,8 - 8,0 ppm (m; 4 H)

10. Unter Feuchtigkeitsausschluß wurde eine Mischung von 323 g (1 Mol) 2-(11-Hydroxy-8-heptadecenyl)-2-oxazolin (technisch) und 131 g Methylen-bis(4-cyclohexylisocyanat) (Desmodur^{(R)} W, Bayer) 48 Stunden auf 80 °C erhitzt. Das Produkt lag nach dem Abkühlen als gelbliche, viskose Flüssigkeit vor.

Die Struktur des Produktes wurde durch das H-NMR-Spektrum belegt: 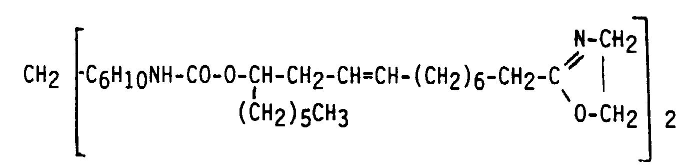
NMR (1 %ig in CDCl₃, delta-Skala)
0,5 - 0,95 ppm (t; 6 H)
0,95 - 1,9 ppm (3m; ca. 64 H)
1,9 - 2,1 ppm (m; 4 H)
2,1 - 2,4 ppm (m; 4 H)
3,3 - 3,5 ppm (m, 2 H)
3,7 - 3,9 ppm (t, 4 H)
4,1 - 4,3 ppm (t; 4 H)
4,3 - 4,6 ppm (m; 2 H)
4,6 - 4,9 ppm (m; 2 H)
5,2 - 5,6 ppm (m; 4 H)

11. Unter Feuchtigskeitsausschluß wurden 337 g (1 Mol) 2-(11-Hydroxy-8-heptadecenyl)-5-methyl-2-oxazolin (technisch) und 125 g (0,5 Mol) 4,4'-Diphenylmethandiisocyanat (Desmodur^{(R)} M, Bayer) unter Rühren bis zur vollständigen Umsetzung für 4 Stunden auf 80 °C erhitzt. Das Produkt erstarrte beim Abkühlen zu einem festen wachsartigen Produkt. Die folgende Struktur wurde durch ein H-NMR-Spektrum belegt. 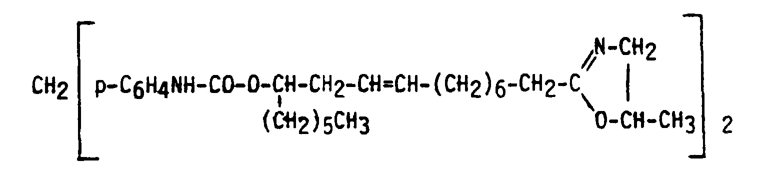
NMR (1 % in CDCl₃, delta-Skala)
0,8 - 1,0 ppm (t; 6 H)
1,1 - 1,8 ppm (2 m; ca. 46 H)
1,9 - 2,4 ppm (1 m + 2 d; 4 + 4 + 4 H)
3,25 - 3,45 ppm (q; 2 H)
3,8 - 4,0 ppm (m + s; 2 + 2 H)
4,5 - 4,75 ppm (m; 2 H)
4,75 - 4,95 ppm (m; 2 H)
5,2 - 5,6 ppm (m; 4 H)
6,7 ppm (s; 2 H)
7,0 - 7,4 ppm (2 d; 8 H)

12. Unter Feuchtigskeitsausschluß wurden 351 g (1 Mol) 2-(11-Hydroxy-8-heptadecenyl)-4,4-dimethyl-2-oxazolin (technisch) und 125 g (0,5 Mol) 4,4'-Diphenylmethandiisocyanat (Desmodur^{(R)} M, Bayer) unter Rühren bis zum vollständigen Umsatz für 8 Stunden auf 80 °C erhitzt. Das Produkt lag nach dem Abkühlen als hellgelbe, viskose Flüssigkeit vor.

Die folgende Struktur des Produktes wurde durch das H-NMR-Spektrum gesichert: 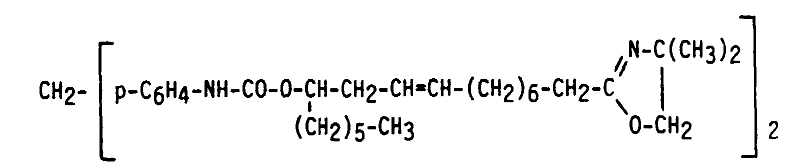
NMR (1 % in CDCl₃, delta-Skala)
0,8 - 1,0 ppm (t; 6 H)
1,1 - 1,45 ppm (m; ca. 48 H)
1,45 - 1,6 ppm (m; 4 H)
1,9 - 2,4 ppm (3 m; 4 + 4 + 4 H)
3,85 - 3,95 ppm (2 s; 4 + 2 H)
4,8 - 4,9 ppm (m; 2 H)
5,2 - 5,6 ppm (m; 4 H)
6,6 - 6,7 ppm (s; 2 H)
7,0 - 7,4 ppm (2 d; 8 H)

13. Unter Feuchtigkeitsausschluß wurde eine Mischung von 351 g (1 Mol) 2-(11-Hydroxy-8-heptadecenyl)-4,4-dimethyl-2-oxazolin (technisch) und 131 g 0,5 Mol 4,4'-Dicyclohexylmethandiisocyanat (Desmodur^{(R)} W, Bayer) 72 Stunden auf 80 °C erwärmt. Das Produkt lag nach dem Abkühlen als gelbliche, viskose Flüssigkeit vor.

Die Struktur des Produktes wurde durch das H-NMR-Spektrum belegt: 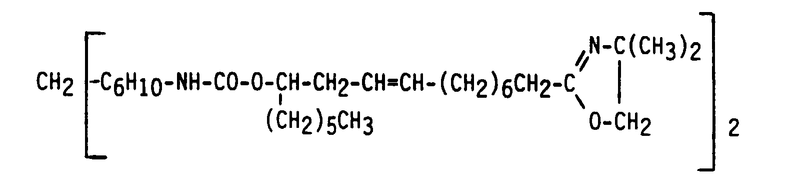
NMR (1 % in CDCl₃, delta-Skala)
0,8 - 2,4 ppm (7 m; ca. 90 H)
3,3 - 3,8 ppm (2 m; 2 H)
3,9 ppm (s; 4 H)
4,3 - 4,85 ppm (2 m; 1 + 3 H)
5,2 - 5,6 ppm (m; 4 H)

14. Unter Feuchtigkeitsausschluß wurde eine Mischung von 157 g (1 Mol) 2-(5-Hydroxypentyl)-2-oxazolin und 111 g (0,5 Mol) Isophorondiisocyanat (VEBA-Chemie) unter Zusatz von 130 mg Dibutylzinndilaurat 8 Stunden auf 100 °C erhitzt. Das Produkt lag nach dem Abkühlen als hochviskose, farblose Substanz vor.

Die Struktur des Produktes wurde durch das H-NMR-Spektrum belegt: 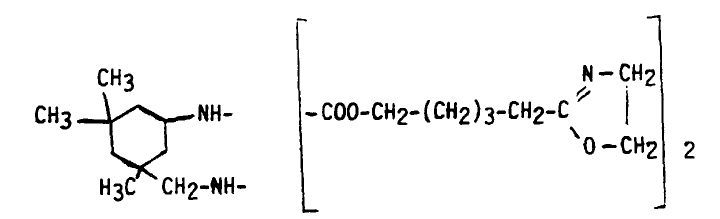
NMR (1 %ig in CDCl₃, delta-Skala)
0,5 - 1,9 ppm (3 m; ca. 27 H)
2,1 - 2,4 ppm (t; 4 H)
2,8 - 3,0 ppm (d; 2 H)
3,1 - 3,55 ppm (2 m; 1 H)
3,7 - 3,9 ppm (t; 4 H)
3,9 - 4,1 ppm (m; 4 H)
4,1 - 4,3 ppm (t; 4 H)
4,4 - 5,0 ppm (2 m; 2 H)

15. Beispiel 14 wurde unter Zusatz von 460 g 2-Nonyl-2-oxazolin als Lösungsmittel wiederholt.

Das erhaltene Gemisch von Mono- und Bisoxazolin lag als farblose niedrigviskose Lösung vor. Das H-NMR stimmte mit dem für ein Gemisch des Monooxazolins mit dem Bisoxazolin im Äquivalentverhältnis 7 : 3 überein.

16. Unter Feuchtigkeitsausschluß wurde eine Mischung von 323 g (1 Mol) 2-(11-Hydroxy-8-heptadecenyl)-2-oxazolin mit 122 g (0,5 Mol) m-Tetramethylxylolylendiisocyanat (m-TMXDI^{(R)}; Cyanamid) 72 Stunden bei 80 °C gerührt. Das Produkt lag nach dem Abkühlen als gelbliche, viskose Flüssigkeit vor.

Die Struktur des Produktes war lt. H-NMR-Spektrum folgende: 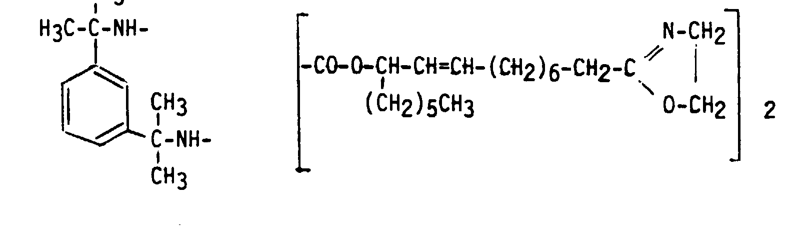
NMR (1 %ig in CDCl₃, delta-Skala)
0,8 - 0,9 ppm (t; 6 H)
1,1 - 1,75 ppm (mm; ca. 52 H)
1,85 - 2,15 ppm (m; 4 H)
2,15 - 2,4 ppm (t; 8 H)
3,7 - 3,9 ppm (t; 4 H)
4,1 - 4,3 ppm (t; 4 H)
4,55 - 4,75 ppm (m; 2 H)
5,05 ppm (s; 2 H)
5,2 - 5,6 ppm (m; 4 H)
7,2 - 7,5 ppm (2 m; 3 + 1 H)

17. Unter Feuchtigkeitsausschluß wurde eine Mischung von 157 g (1 Mol) 2-(5-Hydroxypentyl)-2-oxazolin und 122 g (0,5 Mol) m-Tetramethylxylolylendiisocyanat (m-TMXDI^{(R)}; Cyanamid) in 395 g 2-Heptyl-2-oxazolin als Lösungsmittel 72 Stunden auf 80 °C erwärmt. Das Gemisch von Mono- und Bisoxazolin lag nach dem Abkühlen als schwach gelbliche, niedrigviskose Lösung vor. Das H-NMR-Spektrum entsprach dem eines Gemisches des Mono- und Bisoxazolins im Äquivalentverhältnis 7 : 3 mit folgender Struktur für das Bisoxazolin: 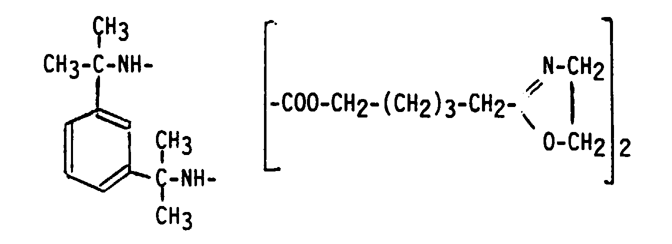
NMR (1 % in CDCl₃, delta-Skala)
0,8 - 0,95 ppm (t; ca.21 H)
1,15 - 1,8 ppm (2 m; ca. 106 H)
2,15 - 2,4 ppm (t; ca. 20 H)
3,7 - 3,9 ppm (t; ca. 20 H)
4,1 - 4,3 ppm (t; ca. 20 H)
5,1 - 5,3 ppm (2 m; 3 H)
7,2 - 7,5 ppm (2 m; 4,5 + 1,5 H)

18. Unter Feuchtigkeitsausschluß wurden 325 g (1 Mol) 2-(11-Hydroxyheptadecyl)-2-oxazolin mit 111 g (0,5 Mol) Isophorondiisocyanat (VEBA-Chemie) unter Zusatz von 220 mg Dibutylzinndilaurat 8 Stunden auf 100 °C erhitzt. Das Produkt erstarrte beim Abkühlen zu einer farblosen, wachsartigen Paste.

Die Struktur des Produktes wurde durch das H-NMR-Spektrum belegt: 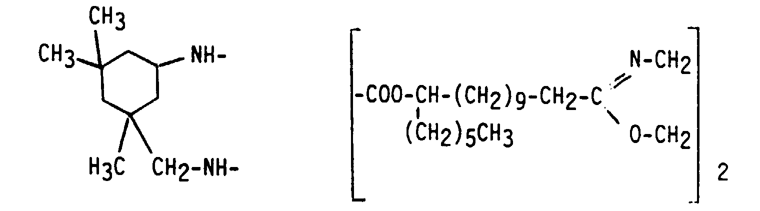
H-NMR (1 %ig in CDCl₃, delta-Skala)
0,8 - 2,0 ppm (6 m; ca. 77 H)
2,2 - 2,3 ppm (t; 4 H)
2,85 - 2,95 ppm (d; 2 H)
3,2 - 3,5 ppm (m; 1 H)
3,7 - 3,9 ppm (t; 4 H)
4,1 - 4,3 ppm (t; 4 H)
4,35 - 4,5 ppm (m; ca. 2 H)
4,6 - 4,8 ppm (m; 2 H)

19. Unter Feuchtigkeitsausschluß wurden 325 g (1 Mol) 2-(11-Hydroxyheptadecyl)-2-oxazolin mit 131 g (0,5 Mol) 4,4'-Dicyclohexylmethandiisocyanat (Desmodur^{(R)} W, Bayer) unter Zusatz von 230 mg Dibutylzinndilaurat 8 Stunden auf 100 °C erhitzt. Das Produkt erstarrte beim Abkühlen zu einem wachsartigen Feststoff

Die Struktur des Produktes wurde durch das H-NMR-Spektrum belegt: 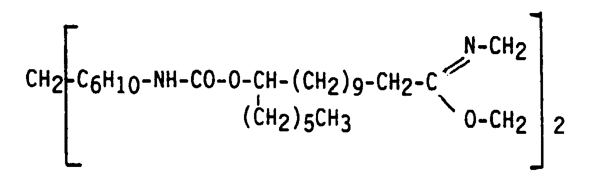
NMR (1 %ig in CDCl₃; delta-Skala)
0,8 - 0,95 ppm (t; 6 H)
0,95 - 2,1 ppm (5 m; ca. 76 H)
2,15 - 2,35 ppm (t; 4 H)
3,25 - 3,65 ppm (m; 2 H)
3,7 - 3,9 ppm (t; 4 H)
4,1 - 4,3 ppm (t; 4 H)
4,3 - 4,55 ppm (m; ca. 2 H)
4,6 - 4,9 ppm (m; 4 H)

## Patentansprüche

1. Verfahren zur Herstellung von Polyiminoethern der allgemeinen Formel I 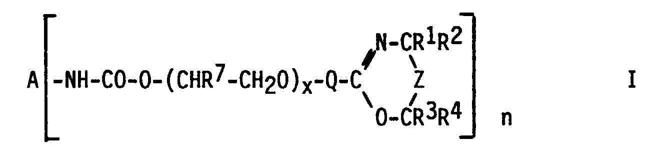 in der A eine aliphatische, aromatische oder araliphatische Gruppe mit 4 bis 40 C-Atomen und n freien Valenzen an verschiedenen C-Atomen darstellt, x einen Wert zwischen 0 und 10 aufweist, n einen der Werte 2, 3 oder 4 hat, Z eine direkte Bindung oder die Gruppe CR⁵R⁶ bedeutet, die Reste R¹ bis R⁶ jeweils unabhängig voneinander H, C₁-C₃ Alkyl bedeuten, wobei wenigstens 4 der Reste R¹ bis R⁶ Wasserstoffatome sind, R⁷ ausgewählt ist aus der Gruppe Wasserstoff, Methyl und Ethyl, und Q eine Phenylengruppe, die durch 1 oder 2 Methylgruppen substituiert sein kann, oder die Gruppe -CHR-M- bedeutet, wobei R ausgewählt ist aus der Gruppe H, Alkyl oder Alkenyl und M ausgewählt ist aus der Gruppe Alkylen, Alkenylen, Arylen und Alkarylen, und die Molekülteile R und M jeweils unabhängig voneinander 0 bis 12 C-Atome aufweisen und durch eine Alkoxygruppe mit 1 bis 3 C-Atomen substituiert sein können, dadurch gekennzeichnet, daß ein Polyisocyanat der Formel A(-NCO)ₙ (II) mit einer wenigstens äquivalenten Menge eines Iminoethers der Formel III 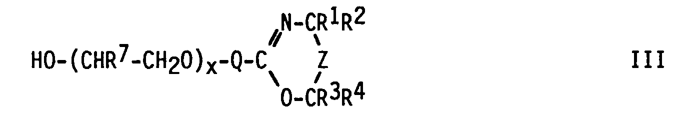 wobei A, Q, R¹ bis R⁷, Z, x und n die oben angegebene Bedeutung haben, unter den für die Umsetzung von Alkoholen mit Isocyanaten üblichen Bedingungen gegebenenfalls unter Zusatz eines üblichen Katalysators und/oder Lösungsmittels, umgesetzt wird.

2. Verfahren zur Herstellung von Polyiminoethern nach Anspruch 1, in dem in den Formeln I und III Q die Gruppe -CHR-M- darstellt.

3. Verfahren zur Herstellung von Bis-, Tris- oder Tetrakisiminoethern nach Anspruch 2, bei dem der Molekülteil -O-CHR-M-wenigstens einmal abgeleitet ist vom 11-Hydroxyheptadecenylrest.

4. Verfahren zur Herstellung von Bisiminoethern gemäß Anspruch 1, bei dem n in den Formeln I und III den Wert 2 hat.

5. Verfahren zur Herstellung von Bisiminoethern gemäß Anspruch 4, bei dem A ausgewählt ist aus der Gruppe 1,6-Hexamethylen; 2,4-Toluylen; 4,4'-Methandiphenyl; 2,4'-Methandiphenyl, 4,4'-Methandicyclohexyl, m-Tetramethylxylolylen und Isophoronylen.

6. Polyiminoether der allgemeinen Formel I 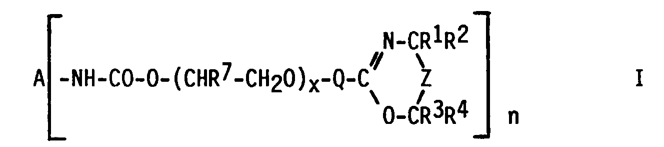 in der A eine aliphatische, aromatische oder araliphatische Gruppe mit 4 bis 40 C-Atomen und n freien Valenzen an verschiedenen C-Atomen darstellt, x einen Wert zwischen 0 und 10 aufweist, n einen der Werte 2, 3 oder 4 hat, Z eine direkte Bindung oder die Gruppe CR⁵R⁶ bedeutet, die Reste R¹ bis R⁶ jeweils unabhängig voneinander H oder C₁-C₃-Alkyl bedeuten, wobei wenigstens 4 der Reste R¹ bis R⁶ Wasserstoffatome sind, R⁷ ausgewählt ist aus der Gruppe Wasserstoff, Methyl und Ethyl, und Q eine Phenylengruppe, die durch 1 oder 2 Methylgruppen substituiert sein kann, oder die Gruppe -CHR-M-bedeutet, wobei R ausgewählt ist aus der Gruppe H, Alkyl oder Alkenyl und M ausgewählt ist aus der Gruppe Alkylen, Alkenylen, Arylen und Alkarylen, und die Molekülteile R und M jeweils unabhängig voneinander 0 bis 12 C-Atome aufweisen und durch eine Alkoxygruppe mit 1 bis 3 C-Atomen substituiert sein können.

7. Polyiminoether nach Anspruch 6, in dem in Formel I Q die Gruppe -CHR-M- darstellt.

8. Bis-, Tris- oder Tetrakisiminoether nach Anspruch 7, bei dem der Molekülteil -O-CHR-M- wenigstens einmal abgeleitet ist vom 11-Hydroxyheptadecenylrest.

9. Bisiminoether gemäß Anspruch 6, bei dem n in Formel I den Wert 2 hat.

10. Bisiminoether gemäß Anspruch 9, bei dem A ausgewählt ist aus der Gruppe 1,6-Hexamethylen; 2,4-Toluylen; 4,4'-Methandiphenyl; 2,4'-Methandiphenyl, 4,4'-Methandicyclohexyl, m-Tetramethylxylolylen und Isophoronylen.

11. Polyiminoether nach einem der Ansprüche 6 bis 10, bei dem Z in Formel I eine direkte Bindung darstellt.

12. Polyiminoether nach Anspruch 11, bei dem R¹ eine Methylgruppe und die Reste R² bis R⁴ Wasserstoff bedeuten.

13. Polyiminoether nach Anspruch 11, bei dem R¹ und R² Methylgruppen und R³ und R⁴ Wasserstoff bedeuten.

14. Verwendung von Polyminioethern erhältlich im Verfahren gemäß einem der Ansprüche 1 bis 5 zur Herstellung von gegebenenfalls vernetzten Polymeren.

## Claims

1. A process for the production of polyiminoethers corresponding to general formula I: 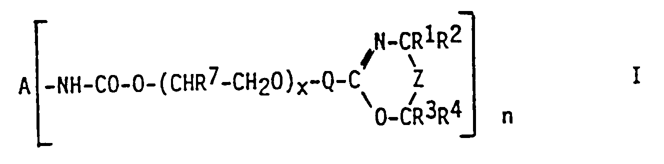 in which A is an aliphatic, aromatic or araliphatic group containing 4 to 40 carbon atoms and n represents free valencies at various carbon atoms, x has a value of 0 to 10, n has a value of 2, 3 or 4, Z is either a direct bond or the group CR⁵R⁶, the substituents R¹ to R⁶ independently of one another are H, C₁-C₃-alkyl or aryl containing up 8 carbon atoms, at least four of the substituents R¹ to R⁶ beeing hydrogen atoms, the substituent R⁷ is selected from the group consisting of hydrogen, methyl and ethyl and Q is a phenylene group which may be substituted by one or two methyl groups or the group -CHR-M-, where R is selected from the group consisting of H, alkyl or alkenyl and M is selected from the group consisting of alkylene, alkenylene, arylene and alkarylene, and the parts R and M of the molecule independently of one another contain 0 to 12 carbon atoms and may be substituted by a C₁₋₃ alkoxy group, characterized in that a polyisocyanate corresponding to the formula A(-NCO)ₙ (II) is reacted with an at least equivalent quantity of an iminoether corresponding to formula III: 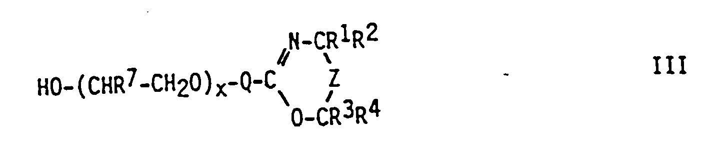 in which A, Q, R¹ to R⁷, Z, x and n are as defined above, under the conditions normally applied for the reaction of alcohols with isocyanates, optionally with addition of a typical catalyst and/or solvent.

2. A process for the production of polyiminoethers as claimed in claim 1, in which Q in formulae I and III is the group -CHR-M-.

3. A process for the production of bis-, tris- or tetrakis-iminoethers as claimed in claim 2, in which the part -O-CHR-M- of the molecule is derived at least once from the 11-hydroxyheptadecenyl radical.

4. A process for the production of bis-iminoethers as claimed in claim 1, in which n in formulae I and III has the value 2.

5. A process for the production of bis-iminoethers as claimed in claim 4, in which A is selected from the group consisting of 1,6-hexamethylene; 2,4-tolylene; 4,4'-methanediphenyl; 2,4'-methanediphenyl, 4,4'-methanedicyclohexyl, m-tetramethyl xyleneylene and isophoroneylene.

6. Polyiminoethers corresponding to general formula I: 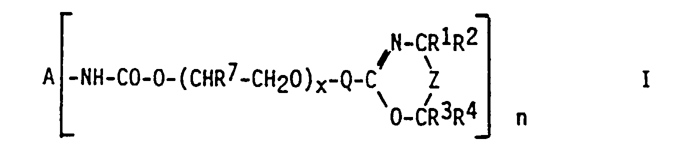 in which A is an aliphatic, aromatic or araliphatic group containing 4 to 40 carbon atoms and n represents free valencies at various carbon atoms, x has a value of 0 to 10, n has a value of 2,3 or 4, Z is either a direct bond or the group CR⁵R⁶, the substituents R¹ to R⁶ independently of one another are H or C₁₋₃ alkyl, at least four the substituents R¹ to R⁶ being hydrogen atoms, the substituent R⁷ is selected from the group consisting of hydrogen, methyl and ethyl and Q is a phenylene group which may be substituted by one or two methyl groups or the group -CHR-M-, where R is selected from the group consisting of H, alkyl or alkenyl and M is selected from the group consisting of alkylene, alkenylene, arylene and alkarylene, and the parts R and M of the molecule independently of one another contain 0 to 12 carbon atoms and may be substituted by a C₁₋₃ alkoxy group.

7. Polyiminoethers as claimed in claim 6, in which Q in formula I is the group -CHR-M-.

8. Bis-, tris- or tetrakis-iminoethers as claimed in claim 7, in which the part -O-CHR-M- of the molecule is derived at least once from the 11-hydroxyheptadecenyl radical.

9. Bis-iminoethers as claimed in claim 6, in which n in formula I has the value 2.

10. Bis-iminoethers as claimed in claim 9, in which A is selected from the group consisting of 1,6-hexamethylene; 2,4-tolylene; 4,4'-methanediphenyl; 2,4'-methanediphenyl, 4,4'-methanedicyclohexyl, m-tetramethyl xyleneylene and isophoroneylene.

11. Polyiminoethers as claimed in any of claims 6 to 10, in which Z in formula I is a direct bond.

12. Polyiminoethers as claimed in claim 11, in which R¹ is a methyl group and the substituents R² to R⁴ are hydrogen.

13. Polyiminoethers as claimed in claim 11, in which R¹ and R² are methyl groups and R³ and R⁴ are hydrogen.

14. The use of the polyiminoethers obtainable by the process claimed in any of claims 1 to 5 for the production of optionally crosslinked polymers.

## Revendications

1. Procédé d'obtention de polyiminoéthers de formule générale I, 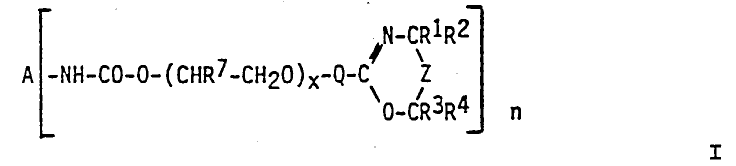 dans laquelle A représente un groupe aliphatique, aromatique ou arylaliphatique ayant de 4 à 40 atomes de carbone, et n valences libres sur différents atomes de carbone, x possède une valeur comprise entre zéro et 10, n a une des valeurs 2, 3 ou 4, Z signifie une liaison directe ou le groupe CR⁵R⁶, les radicaux R¹ à R⁶ respectivement signifient indépendamment l'un de l'autre, H, C₁-C₃-alcoyle ou aryle ayant jusqu'à 8 atomes de carbone, au moins 4 des radicaux R¹ à R⁶ étant des atomes d'hydrogène, R⁷ est choisi dans le groupe hydrogène, méthyle et éthyle, et Q signifie un groupe phénylène, qui peut être substitué par 1 ou 2 groupes méthyle ou signifie le groupe -CHR-M- dans lequel R est choisi dans le groupe formé de H, alcoyle ou alcényle et M est choisi dans le groupe formé d'alcoylène, d'alcénylène, d'arylène et d'alcarylène et les parties de la molécule R et M respectivement, indépendamment l'une de l'autre, possèdent de 0 à 12 atomes de carbone et peuvent être substituées par un radical alcoxy ayant de 1 à 3 atomes de carbone, caractérisé en ce qu'un polyisocyanate de formule A(-NCO)ₙ (II) est mis à réagir avec une quantité au moins équivalente d'un iminoéther de formule III, 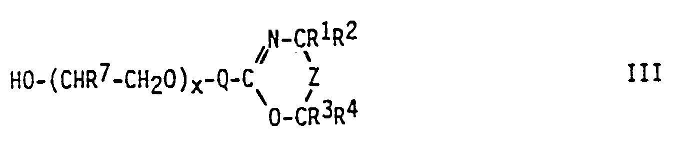 dans laquelle A, Q, R¹ à R⁷, Z, x et n ont les significations mentionnées ci-dessus, dans les conditions habituelles pour la transformation d'alcools avec des isocyanates, le cas échéant en ajoutant un catalyseur et/ou un solvant.

2. Procédé d'obtention de polyiminoéthers selon la revendication 1, dans lequel Q représente dans les formules I et III le groupe -CHR-M.

3. Procédé d'obtention de bis-, tris- ou tétrakisiminoéthers selon la revendication 2, dans lequel la partie de molécule -O-CHR-M est dérivée au moins une fois du radical 11-hydroxyheptadécényle.

4. Procédé d'obtention de bis-iminoéthers conformément à la revendication 1, dans lequel n dans les formules I et III a la valeur 2.

5. Procédé d'obtention de bis-iminoéthers conformément à la revendication 4 dans lequel A est choisi dans le groupe de la 1,6-hexaméthylène, la 2,4-toluylène, la 4,4'-méthanediphényle, la 2,4'-méthanediphényle, le 4,4'-méthanedicyclohexyle, le m-tétraméthylxylolylène et l'isophoronylène.

6. Polyiminoéthers de formule générale I, 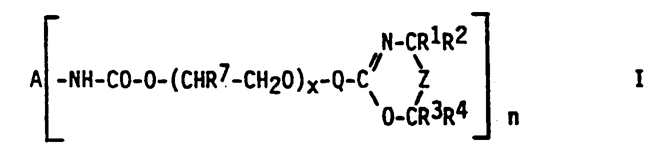 dans laquelle A représente un groupe aliphatique, aromatique ou arylaliphatique ayant de 4 à 40 atomes de carbone, et n représente les valences libres sur différents atomes de carbone, x possède une valeur comprise entre zéro et 10, n a une des valeurs 2, 3 ou 4, Z signifie une liaison directe ou le groupe CR⁵R⁶, les radicaux R¹ à R⁶ respectivement signifient indépendamment l'un de l'autre, H, un alcoyle en C₁ à C₃ au moins 4 des radicaux R¹ à R⁶ étant des atomes d'hydrogène, R⁷ est choisi dans le groupe de l'hydrogène, du méthyle et de l'éthyle, et Q signifie un groupe phénylène, qui peut être substitué par 1 ou 2 groupes méthyle ou signifie le groupe -CHR-M- dans lequel R est choisi dans le groupe formé de H, d'un alcoyle ou alcényle et M est choisi dans le groupe formé d'alcoylène, d'alcénylène, d'arylène et d'alcarylène et les parties de la molécule R et M respectivement, indépendamment l'une de l'autre, possèdent de 0 à 12 atomes de carbone et peuvent être substitués par un groupe alcoxy ayant de 1 à 3 atomes de carbone,

7. Polyiminoéther selon la revendication 6 dans lequel Q dans la formule I représente le groupe -CHR-M.

8. Bis-, tris- ou tétrakisiminoéther selon la revendication 7, dans lequel la partie de molécule -O-CHR-M- est dérivée au moins une fois du radical 11-hydroxy-heptadécényle.

9. Bis-iminoéther conformément à la revendication 6, dans lequel n dans la formule I a la valeur 2.

10. Bis-iminoéther conformément à la revendication 9, dans lequel A est choisi dans le groupe du 1,6-hexaméthylène, 2,4-toluylène, 4,4'-méthanediphényle, 2,4'-méthanediphényle, 4,4'-méthanedicyclohexyle, m-tétraméthylxylolylène et isophoronylène.

11. Polyiminoéther selon l'une des revendications 6 à 10, dans lequel Z représente une liaison directe dans la formule I.

12. Polyiminoéther selon la revendication 11, dans lequel R¹ signifie un groupe méthyle et les radicaux R² et R⁴ signifient de l'hydrogène.

13. Polyiminoether selon la revendication 11, dans lequel R¹ et R² signifient des groupes méthyle et R³ et R⁴ de l'hydrogène.

14. Utilisation de polyiminoéthers accessibles dans le procédé conformément à l'une des revendications 1 à 5, en vue de l'obtention de polymères éventuellement réticulés.
